(19) Europäisches Patentamt
European Patent Office
Office européen des brevets

(11) Publication number: **0 005 064**
**B1**

(12) **EUROPEAN PATENT SPECIFICATION**

(45) Date of publication of patent specification: **10.10.84**

(51) Int. Cl.$^3$: **C 07 D 213/61**

(21) Application number: **79300660.2**

(22) Date of filing: **20.04.79**

(54) **Process for preparing 2,3,5,6-tetrachloropyridine.**

(30) Priority: **24.04.78 US 899675**

(43) Date of publication of application:
**31.10.79 Bulletin 79/22**

(45) Publication of the grant of the patent:
**10.10.84 Bulletin 84/41**

(84) Designated Contracting States:
**BE CH DE FR GB IT NL**

(56) References cited:
**FR-A-1 347 180**
**US-A-3 186 994**
**US-A-3 538 100**
**US-A-3 732 230**

**The file contains technical information submitted after the application was filed and not included in this specification**

(73) Proprietor: **THE DOW CHEMICAL COMPANY**
**Dow Center 2030 Abbott Road Post Office Box 1967**
**Midland Michigan 48640 (US)**

(72) Inventor: **Dietsche, Thomas James**
**30 Roanoke Road**
**Berkeley Alameda California (US)**
Inventor: **Love, Jim**
**3378 Whitehaven Drive**
**Walnut Creek Contra Costa California (US)**

(74) Representative: **Allard, Susan Joyce et al**
**BOULT, WADE & TENNANT 27 Furnival street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 005 064 B1

## Description

The present invention concerns a process for the preparation of 2,3,5,6-tetrachloropyridine.

The chlorinated pyridine derivative of the present invention is a known compound having been previously prepared by a number of processes. This compound has uses, such as pesticides, and is also employed as a chemical intermediate in the preparation of other highly desired herbicide or pesticide products. Previous methods for preparing this compound include those described in U.S. Patent Nos. 3,538,100 and 3,186,994 and the prior art noted therein. According to the '100 patent, pentachloropyridine and 2,3,5,6-tetrachloropyridine (hereinafter referred to for convenience as "Penta" and Tetra" products, respectively,) have been prepared by chlorination of liquid 2,6-dichloropyridine at temperatures of at least about 180°C and in the presence of a metallic halide catalyst. Polychloropyridines, including Penta and Tetra products, are also produced according to the '994 patent by chlorinating a polychloro(trichloromethyl)pyridine reactant in the liquid state at a temperature of at least 160°C, preferably under irradiation with ultraviolet light.

French Patent specification No. 1347180 discloses the chlorination of $\alpha$-picoline in the 6-position at elevated temperatures and also the preparation of other chlorinated 2-(trichloromethyl)pyridine derivatives, such as heptachloropicoline. The production of heptachloropicoline requires low temperatures, i.e., below 120°C and preferably below 105°C. This reaction preferably also proceeds using a catalyst. The use of a low temperature in this reaction is essential to avoid excessive tar formation.

The present invention provides a process for preparing 2,3,5,6-tetrachloropyridine which comprises reacting a liquid chloro-substituted 6-(trichloromethyl)pyridine reactant of the formula:

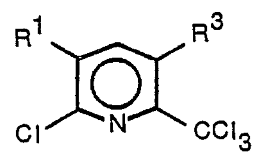

wherein $R^1$ and $R^3$ represent chloro or H, at least one of $R^1$ and $R^3$ being H, with at least an equimolar amount of chlorine in the presence of from 1 to 20 mole percent based on the pyridine reactant, of a Lewis acid type catalyst, the reaction being carried out at a reaction temperature in the range of from 160 to 220°C and a pressure in the range of from atmospheric to 15.4 kg/cm$^2$ gauge (atmospheric to 220 psig) whereby 2,3,5,6-tetrachloropyridine is produced as the major constituent of the reaction product mixture.

The starting material, e.g., 2-chloro-2,3-dichloro- or 2,5-dichloro-6-(trichloromethyl)pyridine, is contacted in the liquid state with chlorine at temperatures of at least about 160°C and at atmospheric or superatmospheric pressure in the presence of a Lewis acid type catalyst.

The process of the present invention is preferably conducted under anhydrous conditions, and is preferably carried out in a continuous, cyclical operation to produce the symmetrical tetrachloropyridine.

In the accompanying drawings, which are more fully referred to in the description following below:

Figure 1 is a diagrammatic sketch showing apparatus used in what is considered to be the best mode known for practising the invention.

Figures 2 and 3 are graphs illustrating results obtained in the batchwise practice of the invention as described in Example 1 — Table A and Example 2 — Table B, respectively.

Figures 4 and 5 are graphs illustrating results obtainable in the practice of the invention on a recycle basis as described in Example 3 and Tables L and M.

Figure 6 is a graph which demonstrates the results of an extended recycle run and effect of tar concentration on production of desired symmetrical tetrachloropyridine.

In carrying out the process of the present invention, gaseous chlorine is passed into a liquid chloro-substituted 6-(trichloromethyl)pyridine starting material at a temperature of at least about 160°C in the presence of a Lewis acid type catalyst. At least an equimolar amount of the chlorine gas reactant is employed with from about 0.3 to about 10 excess molar proportions of chlorine per mole of starting material desirably being employed. The continuous passage of excess chlorine gas through the reaction mixture serves not only to supply a large amount of reactant but to sweep out any carbon tetrachloride or hydrogen chloride by-products. The most suitable rate at which the chlorine gas is fed will vary with the reaction temperature, pressure, reaction mixture volume, etc. An excess amount of from about 0.3 to about 5.0 moles of chlorine per hour is usually employed per mole of chloro-substituted 6-trichloromethyl)pyridine starting reactant.

Representative catalysts include, for example, Lewis acid type catalysts such as metals or metallic halides capable of being converted to covalent metallic chlorides under the conditions of the chlorination reaction of the present invention. Thus, metals themselves such as iron, zinc, aluminum,

and the like can be employed, preferably in the powdered form. Representative covalent metallic chlorides or halides which can be converted to the chloride form include those such as ferric chloride, ferric bromide, aluminum chloride, aluminum bromide, antimony pentachloride, molybdenum pentachloride, tungsten hexachloride, boron trifluoride, titanic chloride, or nickel chloride. As will be understood by those skilled in the art, no equivalency in activity or operability of the catalyst materials is to be inferred. While certain catalysts have been found to provide good results over a short reaction period, for example, at atmospheric pressure, others which may be operable may require long reaction time periods which may not be economically feasible to obtain similar results. Further, certain catalysts may be superior when employed at elevated temperatures and/or pressures. The degree of catalytic activity may also vary depending upon the particular product which is to be produced, the degree of catalyst solubility or miscibility with the starting material, and the use of fixed bed versus slurried catalyst. In any event, those skilled in the art can, by routine experimentation according to the teachings of the specification and numerous examples herein, readily determine the optimum catalyst and amount thereof required for any particular product to be made for any particular set of pressure, temperature or time conditions desired. Catalysts bonded to inert supports or the use of co-catalysts are also contemplated for use in the present invention. Catalysts preferred for use in the present invention include halides of ruthenium, tantalum, tungsten, molybdenun, niobium, aluminum, zinc, and iron. Highly preferred catalysts for use in the present invention include the ferric and aluminum halides, and iron and aluminum metals. A preferred catalyst is ferric chloride. The catalyst are employed in an amount ranging from 1 to 20 mole% by weight based on the amount of chloro-substituted 6-(trichloromethyl)-pyridine starting material. Preferably, a catalyst concentration of from 1.0 to 10 mole% is employed.

While the desired products of the present invention can be obtained by the chlorination, at atmospheric pressure, of 2-chloro-6-(trichloromethyl)-pyridine and other similar reactants in the presence of an effective catalyst at temperatures of from about 160 to about 220°C, it has also been surprisingly found that such products can be obtained in a much more efficient and economical manner if the chlorination reaction is carried out at pressures substantially in excess of atmospheric. Moreover, in the preparation of the tetrachloropyridine, it was surprisingly found that increases in the production of the same were directly correlated to increases in one or more of the pressure, temperature, or catalyst amount parameters. Generally, an increase of 10—15°C in the temperature range has the effect of approximately doubling the reaction rate, while an approximately doubling in the pressure from 7 to 14 kg/cm$^2$ gauge (100 to 200 psig) elicits a similar response. Up to certain levels and with certain catalysts, an approximate doubling of the catalyst amount also has been found to approximately double the reaction rate.

Thus, in carrying out the process of the present invention, illustratively described with respect to 2-chloro-6-(trichloromethyl)pyridine as the starting material, the starting material in molten form is usually added to a reactor previously heated to at least about 100°C and the reactor purged with nitrogen. Catalyst in an amount sufficient to catalyze the reaction is then added and chlorine flow commenced, usually at a sufficient rate to pressure the reactor to about 1 kg/cm$^2$ gauge (15 psig), or more. The temperature of the reactor is then slowly increased to at least about 160°C and the reaction maintained until sufficient amounts of the desired pyridine compounds are obtained. Liquid samples from the reactor and vent gases are periodically taken and analyzed by known methods to monitor the course of the reaction. The reaction is terminated by stopping the heating of the reactor and the flow of chlorine thereto and allowing the reactor pressure to drop to atmospheric. Distillation of the reaction product obtained can then be carried out to obtain the desirable products therefrom and the still bottoms can be recovered and re-used in the process.

The reaction process is generally illustrated below, on a batch-wise basis, for the preparation of the desired product V:

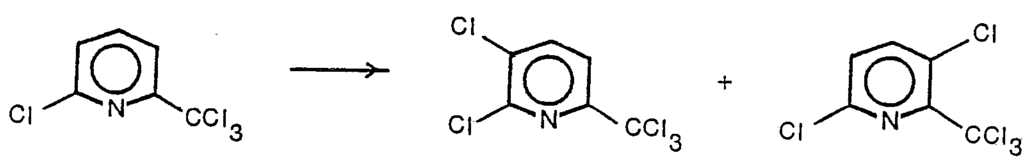

I. II. III.

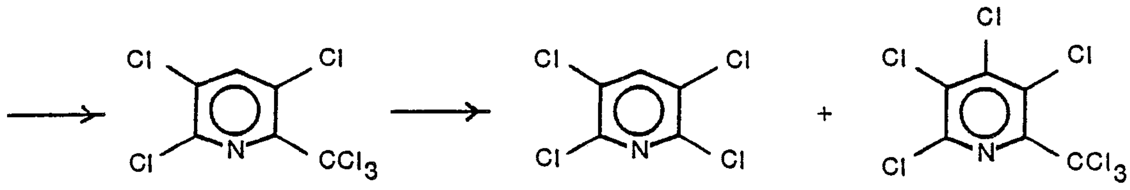

IV. V. VI.

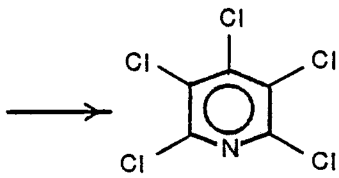

VII.

A small amount of 2,6-dichloropyridine (usually less than 0.1%) is sometimes observed in the initial stage of the reaction. However, the same is apparently quickly converted to 2,3,6-trichloropyridine, which subsequently converts to the desired 2,3,5,6-tetrachloropyridine (V). The amount of 2,3,6-trichloropyridine (also partially derived from some of (III)) formed is also minimal, ranging from about 1% at lower reaction temperatures to about 4% at higher temperatures. During the initial stages of the reaction, conversion of the 2-chloro-6-(trichloromethyl)pyridine (I) is largely to the 2,3-dichloro compound (II) with lesser amounts of the 2,5-dichloro compound (III) being formed. Small amounts (e.g., 4—8% by wt.) of 2,4-dichloro-6-(trichloromethyl)pyridine may sometimes be present as an impurity in starting material (I), and this impurity is converted to 2,4,5-trichloro- and 2,3,4-trichloro-6-(trichloromethyl)pyridine (not illustrated) during the early stages of the reaction and eventually to product (VI). During the formation of the formula (II) and (III) compounds, compounds (IV), (V), (VI) and (VII) are produced in lesser amounts, with the concentrations of products (IV), (V) and (VII) increasing significantly following obtention of peak amounts of compounds (II) and (III). The production of compound (V) continues to increase significantly during the reaction while the concentration of compound (IV) preaks and then begins to diminish. The concentration of compound (VII) continues to increase, although at a lesser rate than compound (V), but will eventually equal and surpass the peak concentration of compound (V) if the reaction is continued for a sufficient period of time.

Those skilled in the art will appreciate that materials (II) or (III) can be derived from sources other than (I) and that they can be used to prepare materials (IV) through (VII), and that material (IV) likewise may be obtainable from other methods known in the art and can be utilized as the starting material to prepare product (V). The use of any one of more of mixtures of these as starting materials is to be understood as being embodiments within the scope of the present invention.

Thus, 2,3- and 2,5-dichloro-6-(trichloro-methyl)pyridine products [(II) and (III)] can be prepared by reacting chlorine and 2-chloro-6-(trichloromethyl)pyridine (I) at atmospheric pressure and at a temperature of at least about 160°C in the presence of a catalyst. The optimum amount of product (II) obtain significant conversion of the starting material thereto is quite long, e.g., substantially in excess products (II) and (III) can be obtained under the conditions noted, the reaction time necessary to obtained significant conversion of the starting material thereto is quite long, e.g., substantially in excess of about 100 hours. Accordingly, for reasons of efficiency and economy, it is preferred that the reaction be carried out at reaction temperatures of at least about 160°C and under pressures substantially in excess of atmospheric, e.g. from 1 to 15.4 kg/cm² gauge (15 to 220 psig) and a catalyst amount of about 2 mole%. In a highly preferred embodiment, the reaction is carried out at temperatures of from about 160°C to about 220°C, pressures of 7 to 15.4 kg/cm² gauge (100 psig to 220 psig) and a catalyst amount of about 4 mole% or more, thereby generally obtaining optimum yields of products (II) and (III). Preferably, a reaction temperature of about 200°C, a reaction pressure of about 14 kg/cm² gauge (200 psig) and a catalyst amount of from about 1 to about 10 mole% are employed. In the latter embodiment, optimum yields of products (II) and (III) can be obtained in a batch reaction in about 10—12 hours. When (II) is the desired product, it is preferred that ruthenium trichloride be employed as the catalyst.

Product (IV) can be prepared from starting material (I) under the same general reaction temperature and pressure ranges as above, with preferred temperatures of from about 160 to about 220°C, pressures of from 7 to 15.4 kg/cm² gauge (100 to 220 psig) and catalyst amounts of from about 2 to about 10 mole% being employed. Most preferably, reaction temperatures of from about 180 to about

190° are employed at reaction pressures of from 13.3 to 14.7 kg/cm² gauge (190 to 210 psig).

The process of the invention is employed to obtain optimum amounts of the tetrachloropyridine compound (V). In such embodiment, the starting material (I) is reacted with chlorine under the same general conditions as set forth above. As noted, the reaction can be conducted at atmospheric pressure, but the reaction time necessary to obtain optimum amounts of the product is extremly long. Therefore, it is preferred that the process be carried out at reaction temperatures of from about 160 to about 220°C, preferably from about 180—210°C, at reaction pressures of from 1 to 15.4 kg/cm² gauge (15 to 220 psig) preferably from 7 to 15 kg/cm² gauge (100 to 220 psig) and at catalyst amounts of from about 1 to about 10 mole%.

While some product can be obtained when operating at the lower ends of said preferred ranges, for example, 170° and 7.7 kg/cm² gauge (110 psig) it was found that certain increases in one of the temperature, pressure, or catalyst amount parameters greatly affected the reaction time needed to obtain optimum amounts of the tetrachloro compound. Generally, it was found the reaction rate about doubled when the reaction pressure was nearly doubled from 7.7 to 14 kg/cm² gauge (120 to 200 psig) the reaction temperature (170°C) and catalyst amount remaining constant. Likewise, a 10—15°C increase in the reaction temperature was found to more than double the reaction rate at a constant pressure of (14 kg/cm² gauge (200 psig) and catalyst concentration.

Similarly, at constant reaction pressure and temperature, for example, 14 kg/cm² gauge (200 psig) and 200°C, the reaction rate was found to be about doubled when the catalyst amount was increased from 2 to 4 mole%. However, the use of larger amounts of catalyst, e.g., generally from about 5 to about 10 mole%, has been found to result in an undesirable build-up of tar by-products in the product still bottoms. This increase in tar build-up is particularly undesirable where the same is recycled with still bottoms to form part of the feed starting material.

Accordingly, reaction temperatures of from about 180 to 210° and reaction pressures of from 13.3 to 14.7 kg/cm² gauge (190 to about 210 psig) are preferred; optimum amounts of the tetra pyridine product of up to about 40—50 weight percent of the reaction product can be obtained under such conditions in reaction periods of from about 40 to 70 hours when using about 2 mole% by weight of catalyst. In a highly preferred embodiment, the reaction temperature is about 200°C, the reaction pressure is about 14 kg/cm² gauge (200 psig) and the catalyst is employed in an amount of from about 1 to about 10 mole%.

It is to be noted that the only constraint placed upon the superatmospheric pressures employed is one of economics, and that pressures in excess of the preferred 190—220 psig 13.3 to 15.4 kg/cm² gauge (190—220 psig) range may be employed. Those skilled in the art will, however, recognize that the cost for pressure equipment to allow operation above 15.4 kg/cm² gauge (220 psig) is greatly increased, and that the cost thereof may exceed any benefits obtained.

The 2-chloro-6-(trichloromethyl)pyridine starting material is known and can be preparared according to the methods taught in U.S. Patent 3,420,833. All of the products (II)—(VII), their physical properties, and methods of analysis therefor are known in the art. The interior surfaces of the reactors and inlets, outlets, conduits, etc., should be of materials which resist corrosion by chlorine and hydrogen chloride. Thus, for example, such surfaces may be lined with glass, carbon, nickel, etc.

The following examples illustrate the liquid phase methods but are not to be construed as limiting the invention. The product distribution in all tables is in terms of weight%. The temperatures are in degrees Centrigrade in the Examples and the Tables.

Example 1

A chlorination reactor comprising a 300 ml flask fitted with a sparge tube connected via a rotometer and needle valve to a chlorine source and a condensor connected to a caustic scrubber was charged with a melt (200 grams) of a 2-chloro-6-(trichloromethyl)pyridine starting material. Catalyst was then charged to the reactor and the reaction mixture warmed to about 200°C at atmospheric pressure with stirring, and chlorine was sparged into the solution at the rate of about 0.1 mole/hr. Samples were removed via a sampling port at 6 hour intervals. The results of operations employing such procedure are set forth below in Table A and illustrated in Figure 2 hereof, the product distribution being in terms of weight percent.

TABLE A

Temperature: 200° Catalyst: 10.0 mole% Fe
Pressure: Atmospheric

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | | 93.3 | 0.3 | — | — | — | — | 6.1 | — |
| 1 | 6 | | 63.0 | 17.5 | 9.2 | 0.7 | 1.9 | 0.1 | 6.5 | 0.8 |
| 2 | 12 | | 32.4 | 35.5 | 18.8 | 2.2 | 3.3 | 0.2 | 5.5 | 2.1 |
| 3 | 18 | | 10.6 | 47.0 | 24.2 | 5.1 | 4.9 | 0.4 | 4.2 | 3.7 |
| 4 | 24 | | 0.6 | 47.6 | 21.7 | 13.9 | 6.6 | 1.1 | 3.1 | 5.3 |
| 5 | 30 | | — | 36.6 | 14.6 | 25.7 | 10.6 | 2.6 | 4.3 | 5.4 |
| 6 | 36 | | — | 30.1 | 9.5 | 32.6 | 14.1 | 3.7 | 5.4 | 4.6 |
| 7 | 42 | | — | 21.4 | 5.9 | 38.0 | 18.9 | 4.8 | 6.8 | 4.1 |
| 8 | 48 | | — | 16.0 | 3.5 | 40.2 | 23.0 | 5.4 | 8.7 | 3.4 |
| 9 | 54 | | — | 10.6 | 1.9 | 40.2 | 27.4 | 5.9 | 11.3 | 2.6 |
| 10 | 60 | | — | 6.3 | 0.8 | 39.3 | 31.4 | 6.2 | 13.9 | 2.0 |
| 11 | 66 | | — | 4.4 | 0.5 | 36.6 | 34.6 | 5.9 | 16.3 | 1.6 |
| 12 | 72 | | — | 2.6 | 0.2 | 33.3 | 36.8 | 6.0 | 19.8 | 1.2 |
| 13 | 78 | | — | 1.4 | 0.1 | 27.9 | 41.3 | 5.5 | 22.5 | 0.9 |

* G.C. peak for VII coincides with peak for 2,4-dichloro-6-(trichloromethyl)pyridine present during early stages of reaction.

** 2,3,4-trichloro-6-(trichloromethyl)pyridine.

Example 2

A two liter, Parr reactor with glass liner and equipped with an air driven stirrer is pre-heated to about 100—125°C and one kilogram of a 2-chloro-6-(trichloromethyl)pyridine starting material is melted and poured into the warm reactor and the desired amount of iron powder catalyst is calculated and added to the starting material melt. The reactor is then sealed, and chlorine, from a gas cylinder placed in a heated and stirred water bath, is delivered to the liquid phase in the heated reactor via a dip-leg. The pressure of the reaction is regulated by heating the chlorine supply cylinder and the $Cl_2$ flow is regulated at a desired level. The rate of vent gases ($Cl_2$ and HCl) is controlled by a pressure regulator. Once the $Cl_2$ flow is commenced to the stirred liquid reaction mass, the reaction is monitored closely until the desired temperature and gas flow is achieved and temperature, pressure and vent gas flow monitored thereafter on a continuous basis. During the course of the reaction, samples of the reaction mass are periodically taken and analyzed. Once the reaction has reached the desired point of completion, the flow of chlorine is stopped and the heating of the reactor is discontinued. Data set forth in the following tables represent the results of several experimental runs using the above-described procedure. Reference to products are as designated in the formulas set forth hereinabove.

TABLE B

Temperature: 200° Catalyst: 2.0 mole% Fe
Pressure: (100 psig) Vent Gas Flow: 50 ml/min.
7.7 $kg/cm^2$ gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 24 | 1.7 | 43.6 | 32.2 | 9.3 | 1.1 | 1.3 | 0.2 | 5.2 | 1.0 |
| 2 | 48 | 1.5 | 13.1 | 51.3 | 12.0 | 2.6 | 6.5 | 0.2 | 4.5 | 2.6 |
| 3 | 72 | 1.4 | 1.1 | 50.2 | 7.6 | 8.9 | 17.6 | 0.7 | 3.4 | 4.6 |
| 4 | 96 | 1.3 | 0.1 | 26.7 | 1.5 | 20.0 | 33.3 | 2.2 | 7.2 | 4.2 |
| 5 | 120 | 1.5 | 0.5 | 17.5 | 0.6 | 16.2 | 42.5 | 2.1 | 11.1 | 3.5 |
| 6 | 144 | 1.5 | 0.7 | 18.3 | 0.5 | 13.1 | 44.8 | 1.9 | 11.8 | 3.8 |

* G.C. peak for VII coincides with peak for 2,4-dichloro-6-(trichloromethyl)pyridine present during early stages of reaction.
** 2,3,4-trichloro-6-(trichloromethyl)pyridine.

TABLE C

Temperature: 200° Catalyst: 2.0 mole% Fe
Pressure: (200 psig) Vent Gas Flow: 50 ml/min.
14 $kg/cm^2$ gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 12 | 1.7 | 46.4 | 32.3 | 8.0 | 1.0 | 0.7 | 0.2 | 5.3 | 0.8 |
| 2 | 24 | 1.7 | 27.2 | 47.1 | 9.5 | 1.9 | 2.6 | 0.3 | 5.1 | 1.5 |
| 3 | 36 | 1.7 | 9.5 | 56.7 | 10.0 | 3.2 | 6.5 | 0.3 | 4.1 | 2.7 |
| 4 | 48 | 1.7 | 1.7 | 57.6 | 8.2 | 6.5 | 12.4 | 0.6 | 3.1 | 4.2 |
| 5 | 60 | 1.7 | 0.4 | 48.2 | 4.1 | 12.7 | 20.1 | 1.1 | 3.6 | 5.0 |
| 6 | 72 | 1.8 | 0.4 | 36.4 | 1.6 | 18.8 | 26.4 | 1.9 | 5.4 | 4.6 |
| 7 | 87 | 1.8 | 0.4 | 20.5 | 0.5 | 20.9 | 36.6 | 2.4 | 8.9 | 3.4 |

* G.C. peak for VII coincides with peak for 2,4-dichloro-6-(trichloromethyl)pyridine present during early stages of reaction.
** 2,3,4-trichloro-6-(trichloromethyl)pyridine.

TABLE D

Temperature: 170° Catalyst: 2.0 mole% Fe
Pressure: (200 psig) Vent Gas Flow: 150 ml/min.
14 kg/cm² gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 8.5 | | 74.2 | 9.2 | 3.4 | 0.5 | 0.3 | 0.2 | 5.1 | 0.2 |
| 2 | 16 | 1.5 | 68.1 | 14.3 | 5.0 | 0.9 | 0.3 | 0.4 | 5.3 | 0.3 |
| 3 | 24 | | 59.3 | 20.4 | 7.1 | 1.3 | 0.3 | 0.5 | 5.0 | 0.5 |
| 4 | 32 | | 49.5 | 28.0 | 9.6 | 1.8 | 0.4 | 0.7 | 4.8 | 0.7 |
| 5 | 40 | 1.7 | 41.6 | 31.7 | 10.9 | 1.9 | 0.7 | 0.8 | 4.5 | 0.8 |
| 6 | 56 | | 25.3 | 42.6 | 15.5 | 3.0 | 1.2 | 1.1 | 3.9 | 1.4 |
| 7 | 68 | | 17.9 | 47.6 | 17.4 | 3.8 | 1.7 | 1.1 | 3.6 | 1.7 |
| 8 | 84 | | 6.3 | 54.4 | 20.0 | 6.1 | 3.3 | 1.3 | 2.7 | 2.6 |
| 9 | 92 | | 2.5 | 53.6 | 19.6 | 8.5 | 4.3 | 1.7 | 2.1 | 3.0 |
| 10 | 104 | 1.6 | 0.5 | 48.6 | 16.8 | 14.8 | 6.0 | 2.1 | 1.6 | 3.4 |
| 11 | 118 | | 0.3 | 39.9 | 12.2 | 24.1 | 8.4 | 3.0 | 2.0 | 3.2 |
| 12 | 128 | | 0.3 | 35.4 | 9.6 | 29.4 | 9.7 | 3.7 | 2.2 | 3.0 |
| 13 | 143 | | 0.1 | 27.2 | 6.3 | 37.6 | 11.7 | 4.7 | 2.8 | 2.8 |
| 14 | 152 | | 0.1 | 22.1 | 4.6 | 41.7 | 13.1 | 5.3 | 3.2 | 2.5 |
| 15 | 184 | 0.5 | 0.1 | 8.5 | 0.9 | 53.9 | 18.0 | 7.6 | 5.8 | 1.5 |
| 16 | 213 | | 0.1 | 3.4 | 0.5 | 55.5 | 19.1 | 8.6 | 10.0 | 0.7 |
| 17 | 237 | | 0.1 | 3.7 | 0.1 | 45.8 | 22.1 | 7.8 | 16.7 | 0.1 |
| 18 | 255 | 0.4 | 0.1 | 3.7 | 0.4 | 34.4 | 29.4 | 5.6 | 21.3 | 0.4 |

* G.C. peak for VII coincides with peak for 2,4-dichloro-6-(trichloromethyl)pyridine present during early stages of reaction.
** 2,3,4-trichloro-6-(trichloromethyl)pyridine.

TABLE E

Temperature: 185° Catalyst: 4.0 mole% Fe
Pressure: (200 psig) Vent Gas Flow: 150 ml/min.
14 $kg/cm^2$ gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 14 | —**** | 34.2 | 37.6 | 12.8 | 1.7 | 0.8 | 0.4 | 4.3 | 1.0 |
| 1E*** | 14 | | 36.2 | 38.7 | 13.2 | 1.8 | 0.8 | 0.5 | 4.3 | 1.0 |
| 2 | 28 | — | 13.7 | 48.4 | 17.0 | 3.6 | 2.6 | 0.6 | 3.2 | 1.9 |
| 2E | 28 | | 15.1 | 51.4 | 17.6 | 3.7 | 2.9 | 0.7 | 3.4 | 2.0 |
| 3 | 47 | — | 0.4 | 43.6 | 9.5 | 18.5 | 13.2 | 1.7 | 2.3 | 3.3 |
| 3E | 47 | | 0.4 | 45.5 | 9.7 | 18.9 | 13.3 | 1.8 | 2.3 | 3.3 |
| 4 | 71 | — | — | 14.4 | 1.1 | 38.0 | 28.5 | 3.9 | 6.7 | 2.2 |
| 4E | 71 | | — | 15.5 | 1.1 | 39.0 | 28.9 | 4.0 | 6.7 | 2.2 |
| 5 | 93 | — | — | 2.3 | 0.1 | 31.7 | 36.5 | 4.3 | 17.3 | 0.6 |
| 5E | 93 | | - | 2.6 | 0.1 | 0.6 | 36.7 | 4.5 | 17.6 | 0.6 |

* G.C. peak for VII coincides with peak for 2,4-dichloro-6-(trichloromethyl)pyridine present during early stages of reaction.
** 2,3,4-trichloro-6-(trichloromethyl)pyridine.
*** E means $FeCl_2$ extracted from sample.
**** Not analyzed.

TABLE F

Temperature: 200° Catalyst: 4.0 mole% Fe
Pressure: (200 psig) Vent Gas Flow: 150 ml/min.
14 $kg/cm^2$ gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 12 | —**** | 12.1 | 47.2 | 16.5 | 3.3 | 4.6 | 0.5 | 3.4 | 2.1 |
| 1E*** | 12 | | 12.5 | 48.4 | 16.7 | 3.4 | 4.7 | 0.5 | 3.4 | 2.2 |
| 2 | 24 | — | 0.6 | 45.3 | 9.9 | 12.1 | 15.9 | 1.0 | 2.7 | 3.6 |
| 2E | 24 | | 0.5 | 48.5 | 10.2 | 12.4 | 16.0 | 1.0 | 2.7 | 3.7 |
| 3 | 40 | — | 0.1 | 12.7 | 0.8 | 28.8 | 35.3 | 3.3 | 8.9 | 2.3 |
| 3E | 40 | | 0.1 | 14.0 | 0.8 | 30.0 | 35.4 | 3.3 | 9.0 | 2.3 |
| 4 | 48 | — | 0.1 | 5.0 | 0.2 | 25.4 | 40.7 | 3.6 | 14.1 | 1.4 |
| 4E | 48 | | 0.1 | 5.8 | 0.4 | 25.9 | 40.9 | 3.5 | 14.1 | 1.4 |

* G.C. peak for VII coincides with peak for 2,4-dichloro-6-(trichloromethyl)pyridine present during early stages of reaction.
** 2,3,4-trichloro-6-(trichloromethyl)pyridine.
*** E means $FeCl_3$ extracted from sample.
**** Not analyzed.

TABLE G

Temperature: 170°  Catalyst: 7.0 mole% Fe
Pressure: (110 psig)  Vent Gas Flow: 50 ml/min.
7.7 kg/cm² gauge

| Sample | Time (hr) | $FeCl_3$ | (I) | (II) | (III) | (IV) | (V) | (VI) | *(VII) | *2,3,4 |
|---|---|---|---|---|---|---|---|---|---|---|
| 0 | 0 | — | 92.2 | 0.3 | — | — | 0.2 | — | 5.7 | — |
| 1 | 24 | 3.8 | 43.1 | 24.9 | 9.7 | 3.3 | 0.3 | 0.5 | 4.0 | 0.7 |
| 2 | 48 | 7.5 | 24.5 | 34.4 | 13.5 | 3.4 | 0.7 | 0.6 | 3.0 | 1.2 |
| 3 | 72 | 4.4 | 11.2 | 45.9 | 17.2 | 4.9 | 1.9 | 0.7 | 2.6 | 2.0 |
| 4 | 9.6 | 4.6 | 4.0 | 47.8 | 17.0 | 8.8 | 4.3 | 1.1 | 1.9 | 2.7 |
| 5 | 120 | 4.3 | 1.4 | 44.4 | 13.1 | 16.4 | 6.8 | 1.7 | 1.7 | 2.9 |
| 6 | 144 | 4.7 | 0.5 | 35.7 | 8.2 | 25.6 | 10.6 | 2.7 | 2.1 | 2.7 |
| 7 | 168 | 2.6 | 0.2 | 25.5 | 4.4 | 38.4 | 13.6 | 4.0 | 2.9 | 2.4 |
| 8 | 192 | 2.2 | 0.2 | 15.3 | 2.0 | 46.3 | 16.4 | 4.9 | 4.1 | 1.8 |
| 9 | 221 | 3.3 | 0.1 | 8.2 | 0.8 | 49.0 | 19.2 | 5.7 | 5.9 | — |
| 10 | 245 | 2.2 | — | 4.7 | 0.4 | 49.4 | 22.5 | 6.1 | 7.9 | — |

* G.C. peak for VII coincides with peak for 2,4-dichloro-6-(trichloromethyl)pyridine present during early stages of reaction.
** 2,3,4-trichloro-6-(trichloromethyl)pyridine.

TABLE H

Temperature: 170°  Catalyst: 7.0 mole% Fe
Pressure: (200 psig)  Vent Gas Flow: 50 ml/min.
14 kg/cm² gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 24 | 4.6 | 29.0 | 35.5 | 15.5 | 2.5 | 0.6 | 0.5 | 3.4 | 1.1 |
| 2 | 48 | 5.2 | 7.1 | 45.3 | 17.0 | 7.8 | 4.0 | 1.0 | 1.8 | 2.4 |
| 3 | 72 | 5.3 | 2.6 | 41.8 | 10.9 | 15.5 | 9.0 | 1.7 | 1.8 | 2.8 |
| 4 | 96 | 5.3 | 2.4 | 38.5 | 7.5 | 17.0 | 12.2 | 2.0 | 2.3 | 2.8 |
| 5 | 120 | 4.8 | 2.1 | 35.3 | 5.9 | 18.0 | 14.3 | 2.1 | 2.7 | 2.9 |

* G.C. peak for VII coincides with peak for 2,4-dichloro-6-(trichloromethyl)pyridine present during early stages of reaction.
** 2,3,4-trichloro-6-(trichloromethyl)pyridine.

TABLE I

Temperature: 185° Catalyst: 7.0 mole% Fe
Pressure: (200 psig) Vent Gas Flow: 150 ml/min.
14 kg/$cm^2$ gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | —**** | 92.2 | 0.3 | — | — | 0.2 | — | 5.7 | — |
| 1 | 12 | — | 3.1 | 46.6 | 21.8 | 9.8 | 2.6 | 0.8 | 1.6 | 2.9 |
| 2 | 18 | — | 1.5 | 42.0 | 16.6 | 15.8 | 6.3 | 1.4 | 1.7 | 3.0 |
| 3 | 24 | — | 0.2 | 33.3 | 9.94 | 24.4 | 12.3 | 2.1 | 2.2 | 2.8 |
| 4 | 31 | — | 0.1 | 19.3 | 3.0 | 37.1 | 21.0 | 3.6 | 3.8 | 2.3 |
| 5 | 42 | — | — | 5.8 | 0.3 | 46.6 | 26.2 | 5.3 | 8.1 | 1.2 |

* G.C. peak for VII coincides with peak for 2,4-dichloro-6-(trichloromethyl)pyridine present during early stages of reaction.
** 2,3,4-trichloro-6-(trichloromethyl)pyridine.
*** Not analyzed.

TABLE J

Temperature: 200° Catalyst: 7.0 mole% Fe
Pressure: (110 psig) Vent Gas Flow: 150 ml/min.
7.7 kg/$cm^2$ gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 12 | 5.3 | 31.9 | 28.1 | 11.6 | 1.3 | 1.0 | 0.2 | 4.1 | 1.1 |
| 2 | 24 | 4.9 | 3.5 | 45.3 | 14.3 | 7.5 | 7.2 | 0.6 | 2.4 | 3.0 |
| 3 | 36 | 5.0 | 0.5 | 26.3 | 3.7 | 24.6 | 19.3 | 2.2 | 4.1 | 2.9 |
| 4 | 48 | 3.5 | 0.2 | 8.3 | 0.6 | 35.5 | 28.5 | 4.4 | 9.3 | 1.7 |
| 5 | 60 | 4.1 | — | 1.0 | 0.1 | 22.5 | 33.8 | 4.3 | 20.2 | 0.4 |
| 6 | 72 | 2.9 | — | 0.2 | — | 12.6 | 29.0 | 3.4 | 39.3 | 0.2 |

* G.C. peak for VII coincides with peak for 2,4-dichloro-6-(trichloromethyl)pyridine present during early stages of reaction.
** 2,3,4-trichloro-6-(trichloromethyl)pyridine.

TABLE K

Temperature: 200° Catalyst: 7.0 mole% Fe
Pressure: (200 psig) Vent Gas Flow: 150 ml/min.
14 $kg/cm^2$ gauge

| Sample | Time (hr) | $FeCl_3$ | (I) | (II) | (III) | (IV) | (V) | (VI) | *(VII) | **2,3,4 |
|---|---|---|---|---|---|---|---|---|---|---|
| 0 | 0 | — | 92.2 | 0.3 | — | — | 0.2 | — | 5.7 | — |
| 1 | 6 | 4.7 | 30.4 | 33.0 | 13.1 | 1.8 | 1.5 | 0.3 | 4.0 | 1.2 |
| 2 | 12 | 4.8 | 4.7 | 46.1 | 14.0 | 5.9 | 7.2 | 0.5 | 2.5 | 2.8 |
| 3 | 18 | 4.9 | 1.9 | 42.9 | 10.0 | 10.4 | 12.1 | 0.8 | 2.5 | 3.3 |
| 4 | 24 | 4.7 | 0.5 | 30.3 | 4.0 | 21.0 | 20.1 | 1.8 | 3.7 | 3.0 |
| 5 | 30 | 3.6 | 0.4 | 15.0 | 1.0 | 30.4 | 30.3 | 3.2 | 7.3 | 2.5 |
| 6 | 36 | 2.9 | 0.3 | 5.8 | 0.3 | 28.2 | 36.3 | 3.6 | 12.1 | 1.5 |
| 7 | 42 | 1.8 | — | 2.4 | 0.1 | 25.3 | 40.8 | 3.9 | 19.8 | 0.9 |

* G.C. peak for VII coincides with peak for 2,4-dichloro-6-(trichloromethyl)pyridine present during early stages of reaction.
** 2,3,4-trichloro-6-(trichloromethyl)pyridine.

The foregoing Tables A—K indicate that varying amounts of the desired product (V) can be prepared in batch reactions at various pressures, temperatures and catalyst concentrations and indicate the effect of varying one or more of said parameters. It is apparent that the product (V) can be obtained in good yields under the general reaction conditions of the present invention. While data on production of several products is noted in the Tables, it will be apparent to those skilled in the art that the batch reactions can be terminated whenever optimum amounts of a desired product have been obtained and such product thereafter recovered.

Thus, as noted in Table A, optimum amounts of products (II) and (III) can be obtained at 200°C and atmospheric pressure after a period of about 18—24 hours when using a high (10 mole%) amount of catalyst, although use of such a high catalyst concentration results in undesirably high levels of tar (in excess of about 10 wt.%) in the product distillation bottoms. However, optimum yields of such products can also be obtained with the use of only about 2.0 mole% catalyst, thus minimizing undesired tar build-up, by increasing the pressure, although a longer reaction period of about 48 hours is required. Table B (see also Figure 3), sample number 2, demonstrates such effect. The effect of further increasing the pressure on the reaction time is shown in Table C, sample 2, about the same optimum amounts of (II) and (III) as obtained in Tables A and B being obtained in a reaction period of about 24.36 hours. Tables D and G indicate that longer reaction periods are also needed to obtain optimum amounts of (II) and (III) when reaction temperatures in the lower end of the operable temperature range are used, even where high pressures and increased catalyst amounts are employed. However, a comparison of data in Tables D and E indicates that the reaction rate is more than doubled as a result of increasing the temperature about 15°C and/or doubling the catalyst concentration. The effect of temperature increase on reaction rate under the same pressure and catalyst conditions as in Table E is readily apparent from the data in Table F (sample number 1) wherein a short reaction period of only about 12 hours result in optimum production of (II) and (III). The effect of doubling the catalyst from about 2 to about 5 mole% under the same temperature and pressure conditions is also readily apparent from a comparison of Table C (samples 3—4) and Table F (sample 1), the reaction rate also being more than doubled.

Similar results are also seen from a comparison of data in Tables H—K, wherein the pressure and temperature parameters where varied while the catalyst amount was kept constant.

As to the preparation of product (IV) from (I), the same general observations as noted above can be drawn from the data of Tables A—K. Optimum operating conditions are indicated by data in Tables I and K, particularly those noted in Table I.

Likewise, the effects of pressure, temperature and catalyst amounts on the preparation of (V) from (I) are as noted above, with the conditions of Tables F and K resulting in the shortest reaction periods for the production of optimum amounts of product (V).

The foregoing examples illustrate the batchwise pratice of the process. The process can, however,

be conducted on a recycle basis. The following example illustrates the preparation of product, starting with substantially pure starting material (I), and illustrates that still bottoms containing original catalyst can be recovered from the distillation of the desired product and recycled, with little loss of catalyst activity, with make-up starting material (I).

Example 3

A pilot-plant chlorination system similar to that employed in Example 2 is utilized, the essential difference being the introduction of chlorine gas to the vapour phase above the liquid starting material (I). In such operation, 118 kg (261 pounds) of a molten 2-chloro-6-(trichloromethyl)pyridine starting material was pumped into a warmed (100°C) glass-lined reactor having a stirrer and the system purged with $N_2$. 0.59 kg. (1.3 pounds) of iron powder, as the catalyst, was added to the reactor and the flow of chlorine gas 7 kg/cm² gauge (100 psig) into the reactor vapor space was commenced. The temperature of the reactor was slowly raised to about 190°C over a period of about 18 hours, and the reactor then pressured to about 14 kg/cm² gauge (200 psig). The reaction temperature during the rest of the reaction period was maintained at about 200°C. Samples are periodically taken and analyzed to monitor the course of the reaction. The reaction is terminated by cutting the heat to the reactor and allowing the pressure to drop to atmospheric. The reactor and lines are then flushed with $N_2$ and the reactor contents removed and subjected to a vacuum for about 12 hours to remove residual $Cl_2$, HCl and $CCL_4$. The contents are then distilled at reduced pressure (about 20 mmHg) and the tetra (V) and penta (VII) products removed over a period of about 20 hours. The still bottoms are collected for recycle experiments. Results obtained as a result of following the above procedures are set forth in the following Table L and are illustrated in Figure 4 hereof:

TABLE L

Temperature: °C* — Catalyst: 2 mole% Fe
Pressure: (200 psig) 14 kg/cm² gauge — Vent Gas Flow: 80—85 Vol. % $Cl_2$

| Sample | Time (hr) | $FeCl_3$ | (I) | (II) | (III) | (IV) | (V) | (VI) | (VII)** |
|---|---|---|---|---|---|---|---|---|---|
| 0 | 0 | — | 87.4 | 1.2 | 0.3 | — | 1.3 | — | 5.7 |
| 1 | 10.2 | — | 67.1 | 15.5 | 6.0 | 0.4 | 0.7 | — | 5.4 |
| 2 | 15.2 | — | 57.1 | 23.3 | 8.7 | 1.2 | 0.5 | — | 5.2 |
| 3 | 18.0 | — | 50.5 | 27.9 | 10.1 | 1.0 | 0.6 | 0.4 | 5.0 |
| 4 | 26.0 | — | 22.5 | 45.0 | 15.4 | 2.2 | 2.5 | 0.4 | 4.3 |
| 5 | 30.0 | — | 10.3 | 52.0 | 16.8 | 3.3 | 5.2 | 0.4 | 3.7 |
| 6 | 34.0 | — | 2.5 | 54.4 | 16.2 | 5.9 | 9.5 | 0.5 | 3.0 |
| 7 | 38.0 | — | 0.4 | 50.0 | 12.6 | 11.5 | 14.2 | 0.8 | 2.7 |
| 8 | 42.0 | — | — | 41.8 | 8.8 | 18.5 | 18.0 | 1.7 | 3.4 |
| 9 | 47.0 | — | — | 30.6 | 5.0 | 25.6 | 23.2 | 2.2 | 4.9 |
| 10 | 52.0 | — | — | 21.8 | 3.0 | 28.7 | 28.4 | 2.6 | 6.7 |
| 11 | 56.0 | — | — | 13.6 | 1.6 | 31.2 | 32.6 | 3.3 | 9.2 |
| 12 | 60.0 | — | — | 7.3 | 0.7 | 31.0 | 36.0 | 3.7 | 12.6 |

* Temperature Profile
0—18 hr. 190°C
18—end 200°

** G.C. peak for VII coincides with peak for 2,4-dichloro-6-(trichloromethyl)pyridine present during early stages of reaction.

A portion (536 grams) of the still bottoms remaining after distillation and removal of products V and VII from the product mixture noted in Sample No. 12 in Table L above was mixed with 536 grams of starting material (I) (about 92% 2-chloro-6-(trichloromethyl)pyridine) to give the recycle seed starting material mix noted at sample 0 in Table M below. No additional catalyst was added, the catalyst being that contained in the recycle still bottoms. The seed starting material was then chlorinated utilizing the equipment and procedures of Example 2. The results obtained are set forth in Table M below and are illustrated in Figure 5.

TABLE M

Temperature: 200°C Catalyst: 1 mole% Fe
Pressure: (200 psig) Vent Gas Flow: 150 ml/min.
14 kg/$cm^2$ gauge

| Sample | Time (hr) | (I) | (II) | (III) | (IV) | (V) | (VI) | *(VII) |
|---|---|---|---|---|---|---|---|---|
| 0 | 0 | 45.1 | 4.8 | 0.1 | 28.4 | — | 4.8 | 12.3 |
| 1 | 9 | 18.5 | 21.7 | 7.9 | 16.6 | 8.6 | 1.9 | 12.1 |
| 2 | 20 | 2.2 | 30.5 | 10.7 | 12.2 | 15.8 | 1.1 | 12.9 |
| 3 | 25 | 0.1 | 25.1 | 6.7 | 18.9 | 20.1 | 1.7 | 13.7 |
| 4 | 33 | — | 17.2 | 3.6 | 22.9 | 24.9 | 2.6 | 16.1 |
| 5 | 36 | — | 11.5 | 1.9 | 24.1 | 28.0 | 3.0 | 18.0 |

* G.C. peak for VII coincides with peak for 2,4-dichloro-6-(trichloromethyl)pyridine present during early stages of reaction.

The foregoing data illustrate the advantage realized in preparing product II by starting with a recycle seed material as well as the ability to recycle catalyst with little loss of activity.

The process of the present invention can also be carried out on a continuous recycle basis to prepare the various products and is the preferred mode for preparing symmetrical tetrachloropyridine (V). Such process comprises the continuous chlorination of a catalyzed liquid phase reaction mass comprising 2-chloro-6-(trichloromethyl)pyridine reaction at temperatures of at least about 160°C and at pressures of from about atmospheric to about 15.4 kg/$cm^2$ gauge (220 psig). The reaction is carried out in a series of reactors for a period of time sufficient to obtain a desired amount of product (V). When such concentration thereof has been reached, the reaction mass is removed from the last reactor and the desired product obtained by fractional distillation of the reaction mass, the still bottoms or a portion thereof being recycled to the first reactor to comprise part of the feed thereto. The overheads can be condensed and the by-products recovered. Preferably the reaction temperature is from about 160 to about 210°C, the reaction pressures are from 13.3 to 15.4 kg/$cm^2$ gauge (190 to 220 psig) and the catalyst amount ranges from about 1.0 to about 10 mole%. A reaction temperature of from about 200°C., a reaction pressure of 14 to 15.4 kg/$cm^2$ gauge (200 to 220 psig) and a catalyst amount of about 1 to about 5 mole % are preferred; temperatures and catalyst amounts in excess of these preferred ranges, while operable, tend to cause an undesired increase in tar build-up, which has a detrimental effect on the production of the desired product.

The continuous cyclical process will be described with reference to the preparation of preferred product (V) under preferred conditions and with the reaction scheme noted in Figure 1, which is presently considered the best mode for the continuous preparation of product (V).

In the flow sheet of Figure 1, the molten 2-chloro-6-(trichloromethyl)pyridine feed containing the catalyst is introduced via line 11 and mixed with recycle still bottoms returning via line 25 and the mixture is pumped (not illustrated) to heated reactor 12 and reacted with vaporized chlorine (vaporizer not illustrated) fed through line 14 to the bottom of reactor 12 from chlorine source 13 to form a chlorinated reaction mixture. The chlorinated reaction mixture flows by gravity overflow from reactor 12 to reactor 16 via line 15 where it is further reacted with chlorine fed to the bottom of reactor 16 through line 17. The chlorinated reaction mixture from reactor 16 is likewise fed through line 18 to reactor 19 and further reacted with chlorine fed to reactor 19 through line 20. The chlorinated reaction mixture is then passed from reactor 19 to distillation unit 22 through line 21, wherein volatile overheads are removed via line 23 and desired product recovered through line 24. The still bottoms are removed from the bottom of unit 22 and transferred as a recycle stream via line 25 to feed line 11. Each

of reactors 12, 16 and 19 are vented (26a, 26b and 26c, respectively) for release of excess $Cl_2$ and other volatile by-products. Monitoring of the vent gases also serves as a means to follow the course of the reaction. Catalyst can be separately added to reactor 12 if so desired (not illustrated) and the amount of tar in the recycle stream can also be controlled by a bleed valve on line 25 (not illustrated). The number of reactors is not considered to be critical and fewer or greater numbers of reactors can be used, the conditions and average residence time for each reactor being accordingly adjusted for the product(s) desired and temperature/pressure conditions employed.

Initially, the reaction can be started by feeding a composition comprising about 90 weight percent or more of the 2-chloro-6-(trichloromethyl)pyridine starting material in the liquid state (at about 75°C) to reactor 12 along with about 2 mole% ferric chloride catalyst and an excess amount of chlorine to provide about 70% $Cl_2$ in the vent gases. The reaction mass is initially heated at about 190°C at a pressure of about 14 kg/cm$^2$ gauge (200 psig) for a period of about 18 hours. Thereafter, the reaction temperature is raised to about 200°C and the catalyzed liquid phase reaction mass passed from reactor 12 through each of succeeding reactors 16 and 19, each maintained at about 200°C and about 14 kg/cm$^2$ gauge (200 psig), with excess $Cl_2$ fed to each. However, since the initial conversion of starting material (I) to products (II) and (III) is a slow process, it is preferred that a seed starting material comprising the catalyst and about 30 weight % 2-chloro-6-(trichloromethyl)pyridine (I), about 40 weight % product (II) and (III) and about 20 weight % (IV), 10% other chlorinated pyridine products be charged to reactor 12 on a continuous basis and the catalyzed liquid reactor mass subjected to chlorination at about 200°C and about 14 kg/cm$^2$ gauge (200 psig) as it passes successively through each of the 3 reactors. While the sparging of the chlorine up through the reaction mass provides agitation thereof, the reaction mass is preferably stirred or recirculated by pump means in each of the reactors. The average residence time in each reactor is from about 18 to about 20 hours.

The reaction mass from reactor 19 is continuously removed to the distillation column 22 wherein the desired product (V) is recovered along with (VII). About 80—90% of the product stream recovered (about 40% by weight of the reaction mass) is desired product (V) while about 10—20% is mostly product (VII). About 60% of the reaction mass comprises by-products HCl and $Cl_2$, which are also separated from the desired products, and the still bottoms and tars (tars being about 1—5 weight % tar based on starting material). The still bottoms and some tar are recycled to reactor 12 along with an appropriate amount of make-up feed and additional catalyst (if needed) to maintain the desired starting material composition. The products obtained from the distillation column may be further purified by fractional distillation or recrystallization procedures.

The foregoing continuous recycle embodiment is illustrated in the following Example 4.

Example 4

A continuous, recycle process utilizing a reactor scheme substantially as set forth in Figure 1 was carried out over a prolonged period to study the effects of tar build-up upon the rate of production of symmetrical tetrachloropyridine (D). Three 3.78 litre (one-gallon) capacity nickel reactors were utilized with the feed starting material, comprising recycle still bottoms and make-up 2-chloro-6-(trichloromethyl)-pyridine (I) starting material (about 30 weight % total of (I) in the feed to the first reactor) and catalyst as needed, being fed at a rate of 3 ml/min. The catalyst amount was maintained at a level of about 1.6 mole%, while the temperature and pressure in each of the reactors was maintained at about 200°C and about 14 kg/cm$^2$ gauge (200 psig), respectively. The average residence time of the reaction mass being chlorinated in each reactor was about 20 hours, the total residence time in the three reactors being about 60 hours before the reaction mass was withdrawn and distilled. The continuous recycle process was continued for a period of 62 days and the weight % of tar versus desired product (V) monitored by analysis of the product material obtained from the last reactor. The results of such operations were plotted and are noted in the graph of Figure 6. As can be seen from Figure 6, the build-up in tar concentration the first 20 days of operation from 1 to about 4 weight % was very detrimental to the production of desired product (V), decreasing the level thereof from a high of nearly 40 weight % to about 20 weight %. Temporarily decreasing the tar level in the recycle stream and hence the starting feed material from a high of 6 weight % to about 1 weight % over a period of about 10 days resulted in increased levels of product (V), the level of product (V) stabilizing at about 25—28 weight % as the tar level in the reaction mass was allowed to again increase to the level of about 3 weight %.

The foregoing example illustrates the continuous, recycle embodiment of the invention and demonstrates the detrimental effect of high tar levels in the reaction mass.

Example 5

In other operations utilizing procedures substantially similar to Examples 1 and 2 hereof, additional catalysts were evaluated for use in the present invention. In such operations, the chlorination of the starting material (I) (about 93% by weight of 2-chloro-6-(trichloromethyl)pyridine) was carried out at atmospheric pressure at a temperature of about 200°C and a catalyst concentration of about 5 mole%.

In one particular set of operations, the weight % of starting material (I) remaining after 20 hours, hours to achieve maximum concentration of products (II) and (IV), and weight % of (V) after 70 hours

were determined with tungsten, molybdenum, tantalum and niobium catalysts and the results are as follows:

TABLE N

| Catalyst | Mole % | I Wt.% | II Hours | IV Hours | V Wt.% |
|---|---|---|---|---|---|
| $WCl_6$ | 5 | <1 | 5 | 55 | 33 |
| $MoCl_5$ | 5 | <1 | 5 | 45 | 28 |
| $MoCl_3$ | 6.1 | <1 | 5—10 | 45 | 20 |
| $MoOCl_4$ | 5 | <1 | 3 | ~40 | 30 |
| $TaCl_5$ | 5 | <1 | 48 | 48 | 17 |
| $NbCl_5$ | 5 | 18 | 45 | >70 | 7 |

In other similar operations, the following area % of (I), (II), (III) and (IV) were obtained with other catalysts after the reaction times indicated:

TABLE O

| Catalyst | Mole % | Hours | I | II | III | IV |
|---|---|---|---|---|---|---|
| — | — | 0 | 93.3 | 0.3 | — | — |
| Aluminum | 5 | 18 | 78.1 | 8.6 | 3.6 | 0.3 |
| *Aluminum | 5 | 24 | 67.2 | 13.3 | 3.8 | 0.7 |
| Zinc (Metal) | 5 | 18 | 88.1 | 3.1 | 1.5 | 0.1 |
| Zinc (Dust) | 5 | 24 | 84.1 | 5.0 | 2.4 | 0.3 |
| **$RuCl_3$ | 6 | 48 | 8.1 | 48.8 | 5.1 | 7.0 |

*1 mole % $I_2$ added.
**Ruthenium Trichloride.

Example 6

Other operations utilizing the procedures of Example 2 were carried out, data from additional runs using aluminum metal (2 mole%) and tantalum pentachloride (2 mole%) being presented in the following Tables P and Q, respectively:

TABLE P

Temperature: 200° Catalyst: 2.0 mole% Al
Pressure: (200 psi) Vent Gas Flow: 150 ml/min.
14 kg/$cm^2$ gauge

| Sample | Time (hr) | (I) | (II) | (III) | (IV) | (V) | (VI) | *(VII) |
|---|---|---|---|---|---|---|---|---|
| 0 | 0 | 92.0 | 0.4 | — | — | 0.1 | — | 6.0 |
| 1 | 5 | 69.4 | 14.1 | 3.1 | 0.4 | 0.2 | 0.1 | 6.1 |
| 2 | 12 | 50.2 | 28.7 | 5.9 | 1.2 | 0.8 | 0.3 | 6.2 |
| 3 | 18 | 38.6 | 36.5 | 6.7 | 1.4 | 1.4 | 0.3 | 6.1 |
| 4 | 24 | 29.8 | 44.0 | 7.9 | 1.6 | 2.4 | 0.3 | 6.0 |
| 5 | 36 | 10.6 | 53.6 | 8.9 | 3.5 | 6.7 | 0.4 | 4.8 |
| 6 | 48 | 3.7 | 56.8 | 7.4 | 4.0 | 11.3 | 0.3 | 3.6 |
| 7 | 60 | 0.3 | 46.8 | 3.8 | 10.4 | 18.0 | 1.1 | 3.5 |
| 8 | 68 | 0.3 | 40.8 | 2.2 | 13.2 | 23.4 | 1.4 | 4.6 |

* G.C. peak for VII coincides with peak for 2,4-dichloro-6-(trichloromethyl)pyridine present during early stages of reaction.

TABLE Q

Temperature: 200° Catalyst: 2 mole% $TaCl_5$
Pressure: (200 psi) Vent Gas Flow: 150 ml/min.
14 kg/$cm^2$ gauge

| Sample | Time (hr) | (I) | (II) | (III) | (IV) | (V) | (VI) | *(VII) |
|---|---|---|---|---|---|---|---|---|
| 0 | 0 | 92.2 | 0.3 | — | — | 0.2 | — | 5.7 |
| 1 | 4 | 51.7 | 23.0 | 8.6 | 1.0 | 0.5 | 0.2 | 5.1 |
| 2 | 11 | 19.8 | 42.5 | 13.2 | 2.5 | 1.6 | 0.2 | 3.4 |
| 3 | 18 | 6.9 | 50.5 | 14.2 | 3.7 | 4.5 | 0.2 | 2.7 |
| 4 | 24 | 0.8 | 51.2 | 12.4 | 7.8 | 9.1 | 0.5 | 2.2 |
| 5 | 30 | — | 39.2 | 6.3 | 19.8 | 14.0 | 1.5 | 2.7 |
| 6 | 36 | — | 25.2 | 2.4 | 27.4 | 20.1 | 2.2 | 4.4 |
| 7 | 42 | — | 14.0 | 0.7 | 31.6 | 24.9 | 3.0 | 7.2 |
| 8 | 46 | — | 9.0 | 0.1 | 29.8 | 31.9 | 3.0 | 10.6 |

* G.C. peak for VII coincides with peak for 2,4-dichloro-6-(trichloromethyl)pyridine present during early stages of reaction.

**Claims**

1. A process for preparing 2,3,5,6-tetrachloropyridine which comprises reacting a liquid chloro-substituted 6-(trichloromethyl)pyridine reactant of the formula:

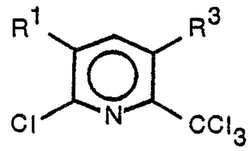

wherein $R^1$ and $R^3$ represent chloro or H, at least one of $R^1$ and $R^3$ being H, with at least an equimolar amount of chlorine in the presence of from 1 to 20 mole percent, based on the pyridine reactant, of a Lewis acid type catalyst, the reaction being carried out at a reaction temperature in the range of from 160 to 220°C and a pressure in the range of from atmospheric to 15.4 kg/cm² gauge (atmospheric to 220 psig), whereby 2,3,5,6-tetrachloropyridine is produced as the major constituent of the reaction product mixture.

2. A process as claimed in claim 1 wherein the catalyst is employed in an amount of from 1 to 10 mole percent, based on the pyridine reactant.

3. A process as claimed in claim 1 or claim 2 wherein the pressure is in the range of from 1 to 15.4 kg/cm² (15 to 220 psig).

4. A process as claimed in any one of the preceding claims wherein the reaction is carried out at a temperature in the range of from 180 to 210°C under pressure in the range of from 7 to 15.4 kg/cm² gauge (100 to 220 psig).

5. A process as claimed in any one of the preceding claims wherein the reaction is carried out at a temperature of about 200°C and a reaction pressure of 14 kg/cm² gauge (200 psig) and the catalyst is employed in an amount of from 1 to 10 mole percent, based on the pyridine reactant.

6. A process as claimed in any one of the preceding claims wherein the catalyst is ferric chloride, ferric bromide, aluminium chloride, aluminium bromide, antimony pentachloride, molybdenum pentachloride, tungsten hexachloride, boron trifluoride, titanic chloride or nickel chloride.

**Patentansprüche**

1. Verfahren zur Herstellung von 2,3,5,6-Tetrachlorpyridin, dadurch gekennzeichnet, daß man ein flüssiges chlorsubstituiertes 6-(Trichlormethyl)-pyridin der Formel

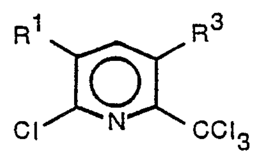

worin $R^1$ und $R^3$ Chlor oder H bedeuten, mindestens einer der Reste $R^1$ und $R^3$ H ist, mit mindestens einer äquimolaren Menge Chlor in Gegenwart von 1 bis 20 Mol-%, bezogen auf den Pyridin-Reaktanten, eines Katalysators, vom Lewis-Säure-Typ umsetzt, wobei die Reaktion bei einer Temperatur im Bereich von 160 bis 220°C und einem Druck im Bereich von Atmosphärendruck bis 15,4 kg/cm² Manometerdruck (atmosphärischer Druck bis 220 psig) durchgeführt wird, und 2,3,5,6-Tetrachlorpyridin als Hauptbestandteil der Reaktionsproduktmischung gebildet wird.

2. Verfahren nach Anspruch 1, dadurch gekennzeichnet, daß der Katalysator in einer Menge von 1 bis 10 Mol-%, bezogen auf den Pyridin-Reaktanten, verwendet wird.

3. Verfahren nach Anspruch 1 oder 2, dadurch gekennzeichnet, daß der Druck im Bereich von 1 bis 15,4 kg/cm² (15 bis 220 psig) ist.

4. Verfahren nach einem der vorhergehenden Ansprüche, dadurch gekennzeichnet, daß die Reaktion bei einer Temperatur im Bereich von 180 bis 210°C unter einem Druck im Bereich von 7 bis 15,4 kg/cm² Manometerdruck (100 bis 220 psig) durchgeführt wird.

5. Verfahren nach einem der vorhergehenden Ansprüche, dadurch gekennzeichnet, daß die Reaktion bei einer Temperatur von ca. 200°C und einem Reaktionsdruck von 14 kg/cm² Manometerdruck (200 psig) durchgeführt wird und der Katalysator in einer Menge von 1 bis 10 Mol-%, bezogen auf den Pyridin-Reaktanten, verwendet wird.

6. Verfahren nach einem der vorhergehenden Ansprüche, dadurch gekennzeichnet, daß der Katalysator Eisen(III)-chlorid, Eisen(III)-bromid, Aluminiumchlorid, Aluminiumbromid, Antimonpentachlorid, Molybdänpentachlorid, Wolframhexachlorid, Bortrifluorid, Titan(IV)-chlorid oder Nickelchlorid ist.

## Revendications

1. Procédé de préparation de 2,3,5,6-tétrachloropyridine, qui consiste à faire réagir un réactif 6-(trichlorométhyl)-pyridine chloro-substituée liquide de formule:

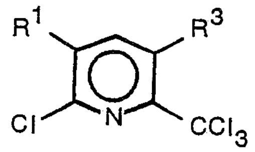

dans laquelle $R^1$ et $R^3$ répresentent le radical chloro ou H, au moins l'un des radicaux $R^1$ et $R^3$ étant H, avec au moins une quantité équimolaire de chlore en présence de 1 à 20% molaire, sur la base du réactif pyridine, d'un catalyseur du type acide de Lewis, la réaction étant réalisée à une température de réaction comprise entre 160 et 220°C, et sous une pression comprise entre la pression atmosphérique et 1509 kPa rel. (entre la pression atmosphérique et 220 psig), la 2,3,5,6-tétrachloropyridine étant produite en tant que constituant principal du mélange de produits de réaction.

2. Procédé selon la revendication 1, dans lequel le catalyseur est utilisé en une quantité de 1 à 10% molaire sur la base du réactif pyridine.

3. Procédé selon la revendication 1 ou 2, dans lequel la pression est comprise entre 98 et 1509 kPa rel. (15 à 220 psig).

4. Procédé selon l'une quelconque des revendications 1 à 3, dans lequel la réaction est réalisée à une température comprise entre 180 et 210°C sous une pression comprise entre 686 et 1509 kPa rel. (110 à 220 psig).

5. Procédé selon l'une quelconque des revendications 1 à 4, dans lequel la réaction est réalisée à une température d'environ 200°C et sous une pression de réaction de 1372 kPa rel. (200 psig), le catalyseur étant utilisé en une quantité de 1 à 10% molaire sur la base de réactif pyridine.

6. Procédé selon l'une quelconque des revendications 1 à 5, dans lequel le catalyseur est le chlorure ferrique, le bromure ferrique, le chlorure d'aluminium, le bromure d'aluminium, le pentachlorure d'antimoine, le pentachlorure de molybdène, l'hexachlorure de tungstène, le trifluorure de bore, le chlorure titanique ou le chlorure de nickel.

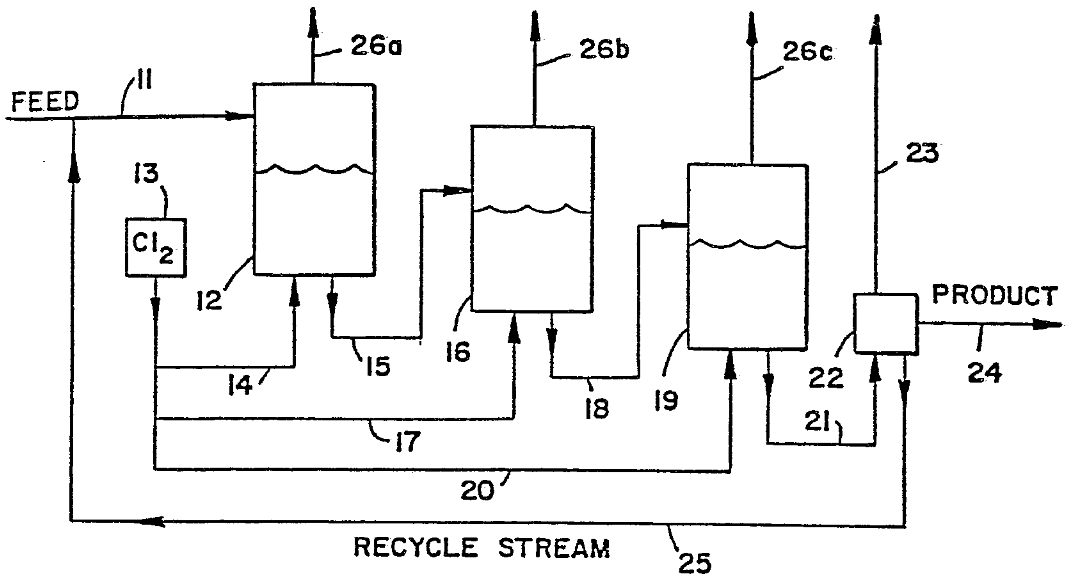
FEED
11
13
$Cl_2$
12
26a
26b
26c
23
14
15
16
17
18
19
20
21
22
PRODUCT
24
RECYCLE STREAM
25

FIG_1

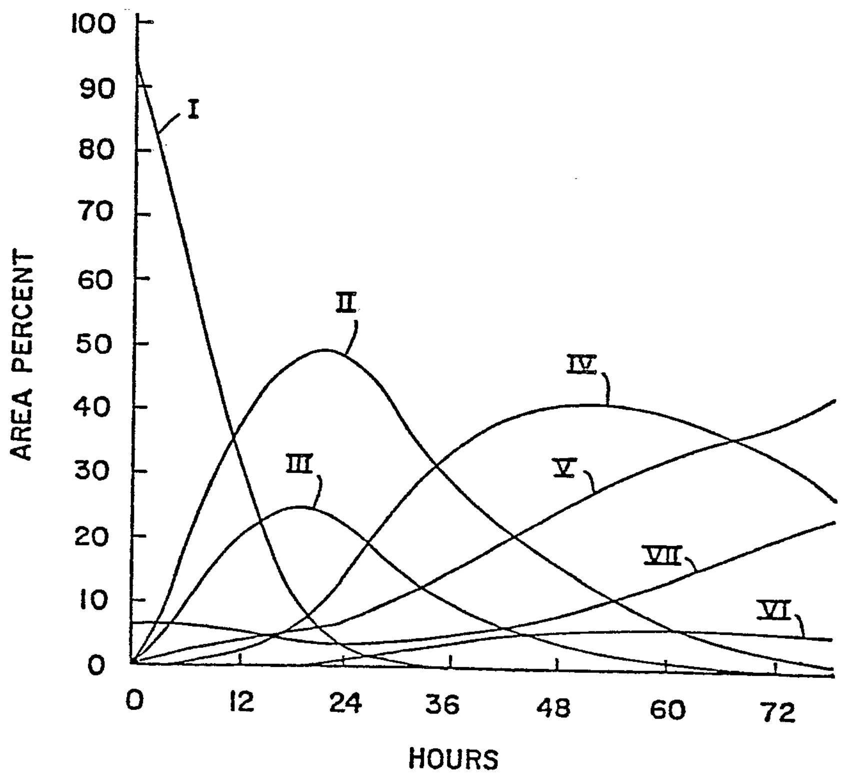
100
90
80
70
60
50
40
30
20
10
0
AREA PERCENT
I
II
III
IV
V
VI
VII
0
12
24
36
48
60
72
HOURS

FIG_2

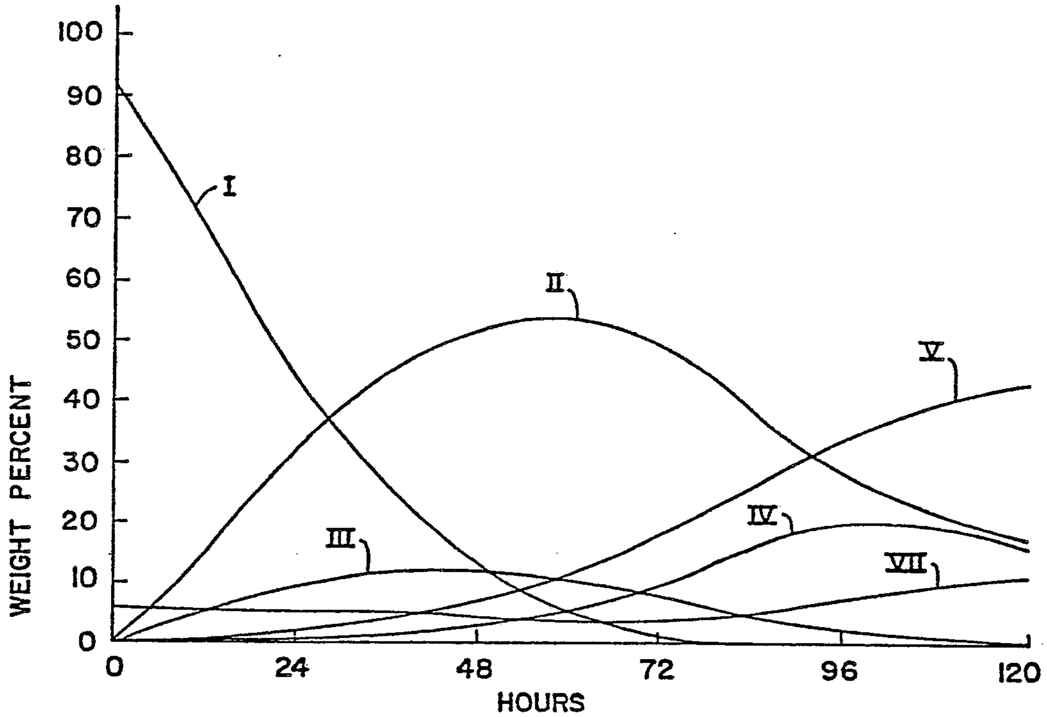
100
90
80
70
60
50
40
30
20
10
0
WEIGHT PERCENT
0
24
48
72
96
120
HOURS
I
II
III
IV
V
VII

FIG _ 3

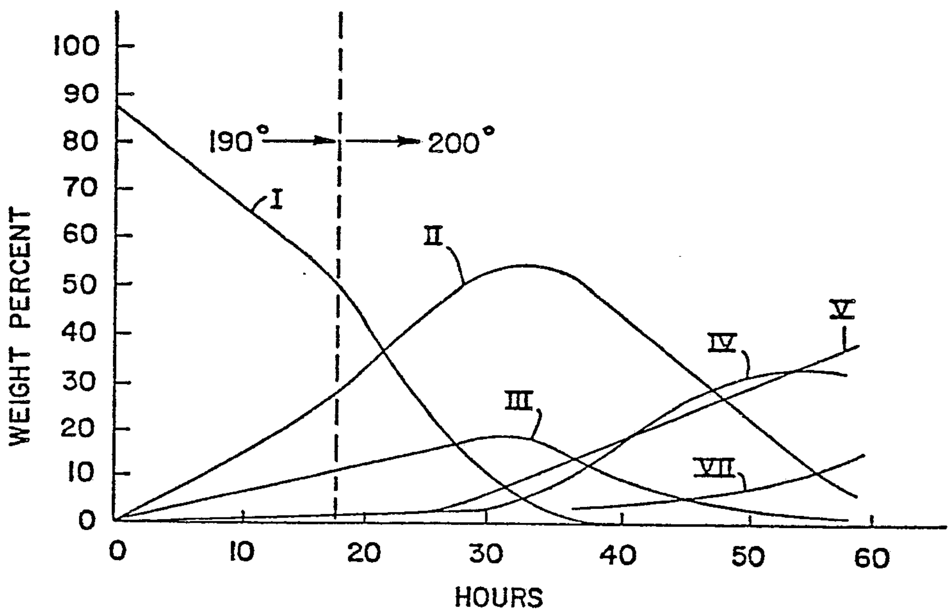
100
90
80
70
60
50
40
30
20
10
0
WEIGHT PERCENT
190°
200°
0
10
20
30
40
50
60
HOURS
I
II
III
IV
V
VII

FIG _ 4

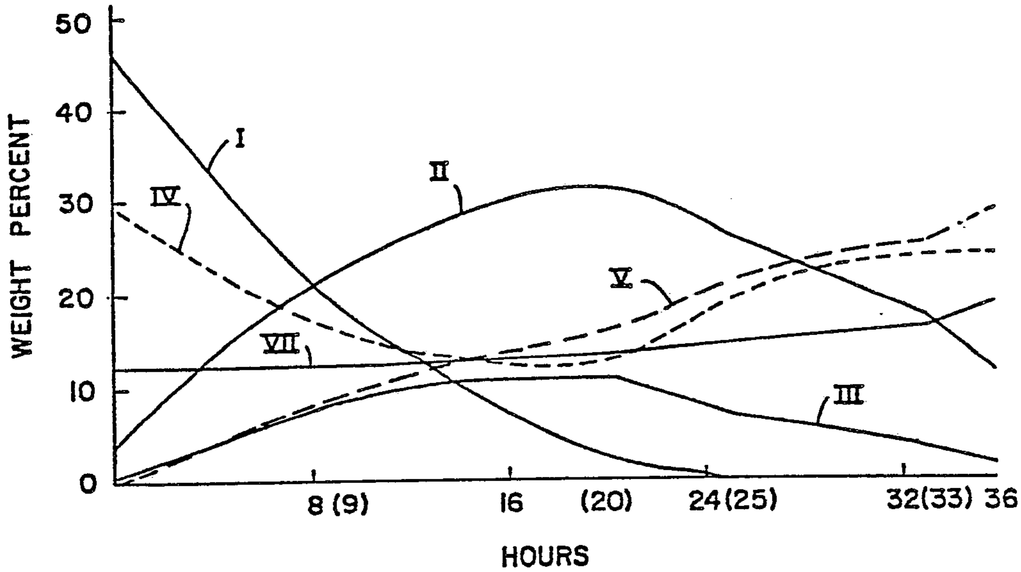

50
40
30
20
10
0
WEIGHT PERCENT
I
II
III
IV
V
VII
8 (9)
16
(20)
24(25)
32(33)
36
HOURS


FIG _ 5

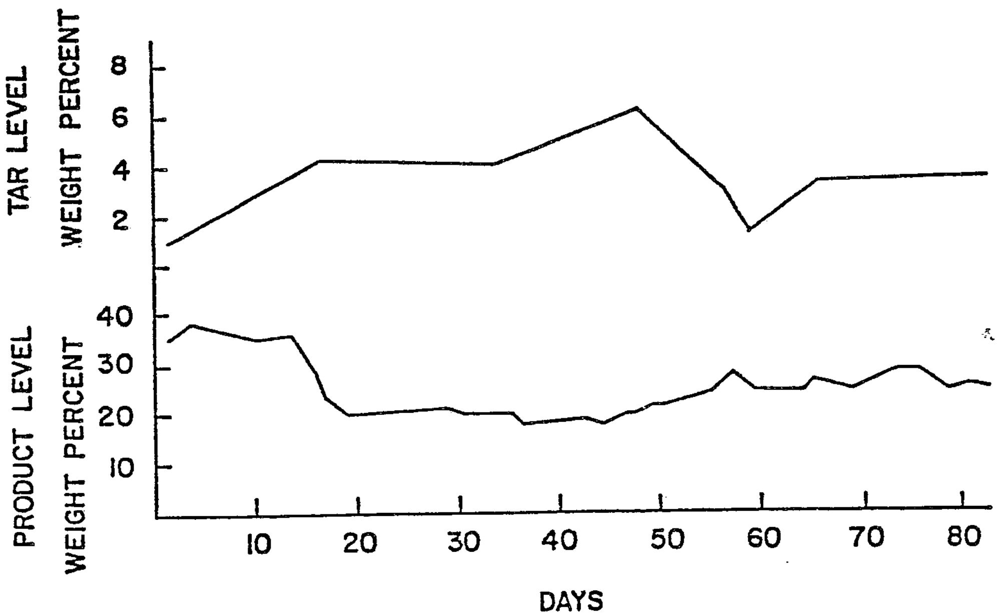

TAR LEVEL
WEIGHT PERCENT
8
6
4
2
PRODUCT LEVEL
WEIGHT PERCENT
40
30
20
10
10
20
30
40
50
60
70
80
DAYS


FIG _ 6